(19) **Europäisches Patentamt / European Patent Office / Office européen des brevets**

(11) **EP 4 046 977 A1**

(12) # EUROPEAN PATENT APPLICATION
published in accordance with Art. 153(4) EPC

(43) Date of publication:
**24.08.2022 Bulletin 2022/34**

(21) Application number: **20875726.0**

(22) Date of filing: **16.10.2020**

(51) International Patent Classification (IPC):
**C04B 35/486** (2006.01)    **A61C 5/77** (2017.01)
**A61C 13/083** (2006.01)    **A61K 6/818** (2020.01)

(52) Cooperative Patent Classification (CPC):
**A61C 5/77; A61C 13/083; A61K 6/818;
C04B 35/486**

(86) International application number:
**PCT/JP2020/039157**

(87) International publication number:
**WO 2021/075564 (22.04.2021 Gazette 2021/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.10.2019  JP 2019190407**

(71) Applicant: **Kuraray Noritake Dental Inc.
Kurashiki-shi, Okayama 710-0801 (JP)**

(72) Inventor: **KATO, Shinichiro
Miyoshi-shi, Aichi 470-0293 (JP)**

(74) Representative: **D Young & Co LLP
120 Holborn
London EC1N 2DY (GB)**

(54) **ZIRCONIA MOLDED ARTICLE SUITABLE FOR DENTAL USE**

(57)    The present invention provides a zirconia molded body and a dental mill blank with which a decrease of the translucency of a zirconia sintered body upon firing can be reduced even when the cooling solvent used for wet processing of the zirconia molded body or dental mill blank by a dental CAD/CAM system is contaminated, and that enable more convenient fabrication of a zirconia sintered body. The present invention relates to a zirconia molded body comprising zirconia; a stabilizer capable of inhibiting a phase transformation of zirconia; and a binder, and having an open porosity of 25% or less.

EP 4 046 977 A1

**Description**

TECHNICAL FIELD

The present invention relates to a zirconia molded body.

BACKGROUND ART

The dental CAD/CAM system is a technology available in dentistry to make a dental prosthesis to be installed in the oral cavity of a patient, whereby silicate glass-a highly translucent material with desirable aesthetics-or a high-strength ceramic material such as zirconia is worked into a shape that fits the affected area of a patient's tooth, and fired into the product dental prosthesis. Dental zirconia is a type of zirconia material available for this purpose.

Earlier types of dental zirconia had high strength but were very opaque in quality. In response to demands from patients, today's dental zirconia has a level of translucency comparable to that of natural teeth. Fabrication of all-zirconia dental prostheses is usually performed at dental laboratories. However, it is becoming increasing popular to more conveniently make such dental prostheses at the dental clinic by using a more quickly firable zirconia pre-sintered body.

Typically, milling machines used in the dental CAD/CAM system employ at least one of wet and dry processing while other types of milling machines are adapted to both wet and dry processing, as described in Non Patent Literatures 1 and 2. A glass material such as silicate glass is processed by wet processing that circulates a processing liquid to prevent the burning of work tools due to the high density of such glass materials. In the case of dental zirconia used in the form of a pre-sintered body, at least one of wet and dry processing is selected. In wet processing, there are cases where the cooling solvent is shared by a glass material and a zirconia material, and this is known to cause a zirconia sintered body to turn white (see Patent Literature 1). Usually, this necessitates the cooling solvent to be changed after wet processing of a glass material, before processing a zirconia material by wet processing.

Patent Literature 1 discloses a dental zirconia pre-sintered body that can prevent such whitening of a zirconia sintered body. In Patent Literature 1, a glass component that dissolves into the cooling solvent is pointed out as a cause of whitening of a zirconia sintered body. This patent document discloses that permeation of a contaminated cooling solvent into a pre-sintered body can be prevented by impregnating the pre-sintered body with a specific impregnation material, and that this inhibits whitening of a zirconia sintered body even in a wet process sharing the cooling solvent with a glass material. However, fabrication of the zirconia pre-sintered body disclosed in Patent Literature 1 involves pressing a raw-material zirconia granule into a zirconia molded body, and pre-sintering the zirconia molded body in a retained temperature range of typically 800 to 1,200°C to provide the strength that can withstand the process. Another drawback is that the fabrication process is very laborious because the pre-sintered body needs to be processed with a specific impregnation material after fabrication.

CITATION LIST

Patent Literature

Patent Literature 1: JP 2019-108289 A

Non Patent Literature

Non Patent Literature 1:ASAHIROENTGEN IND. CO., LTD., Product Information, [online], [search date: August 19, 2019], Internet <URL http://www.asahi-xray.co.jp/products/ceramill-motion2>
Non Patent Literature 2: Dentsply Sirona, CEREC Milling Units, [online], [search date: October 7, 2019], Internet <URL https://www.dentsplysirona.com/ja-jp/explore/cerec/produce-with-cerec.html>

SUMMARY OF INVENTION

Technical Problem

There accordingly is a need for more convenient fabrication of a dental mill blank that does not involve whitening even when the same cooling solvent is used for wet processing of both glass material and zirconia material by a dental CAD/CAM system.

An object of the present invention is to provide a zirconia molded body and a dental mill blank with which a decrease of the translucency of a zirconia sintered body upon firing can be reduced even when the cooling solvent used for wet processing of the zirconia molded body or dental mill blank by a dental CAD/CAM system is contaminated, and that enable more convenient fabrication of a zirconia sintered body.

Solution to Problem

The present inventor conducted intensive studies to find a solution to the foregoing issues, and found that such a dental mill blank can be conveniently fabricated with a zirconia molded body having a specific open porosity. The present invention was completed on the basis of this finding.

Specifically, the present invention includes the following.

[1] A zirconia molded body

comprising

zirconia;
a stabilizer capable of inhibiting a phase transformation of zirconia; and
a binder, and

having an open porosity of 25% or less.

[2] The zirconia molded body according to [1], wherein the open porosity is 5% or more.
[3] The zirconia molded body according to [1] or [2], wherein the binder is present in an amount of 3 to 10 mass% relative to a total mass of the zirconia, the stabilizer, and the binder.
[4] The zirconia molded body according to [3], wherein the binder is present in an amount of 5 to 8 mass%.
[5] The zirconia molded body according to any one of [1] to [4], wherein the zirconia molded body has a water absorbency of 7% or less.
[6] The zirconia molded body according to any one of [1] to [5], wherein the zirconia is predominantly a monoclinic crystal system.
[7] The zirconia molded body according to any one of [1] to [6], wherein at least a part of the stabilizer is undissolved in the zirconia as a solid solution.
[8] The zirconia molded body according to any one of [1] to [7], wherein the stabilizer is yttria.
[9] The zirconia molded body according to [8], wherein the yttria is present in an amount of 3 mol% to 7.5 mol% relative to a total mole of the zirconia and the yttria.
[10] The zirconia molded body according to any one of [1] to [9], wherein the binder is at least one selected from the group consisting of polyvinyl alcohol, methyl cellulose, carboxymethyl cellulose, polyvinyl butyrate, a wax, and an acrylic binder.
[11] The zirconia molded body according to any one of [1] to [10], wherein the zirconia molded body has a biaxial flexural strength of 7 to 30 MPa.
[12] A dental mill blank comprising a zirconia molded body of any one of [1] to [11].

Advantageous Effects of Invention

According to the present invention, a zirconia molded body and a dental mill blank can be provided with which a decrease of the translucency of a zirconia sintered body upon firing can be reduced even when the cooling solvent used for wet processing of the zirconia molded body or dental mill blank by a dental CAD/CAM system is contaminated, and that enable more convenient fabrication of a zirconia sintered body. A zirconia molded body of the present invention can produce a zirconia sintered body having a level of translucency comparable to that of a zirconia sintered body produced from a zirconia pre-sintered body by dry processing and main firing. This makes it possible to reduce defects such as cracking, and provide a zirconia sintered body for dentistry having excellent translucency and other desirable aesthetic qualities, and that is useful as a dental prosthesis.

DESCRIPTION OF EMBODIMENTS

A zirconia molded body of the present invention comprises zirconia; a stabilizer capable of inhibiting a phase transformation of zirconia; and a binder, and has an open porosity of 25% or less.
A zirconia molded body of the present invention has an open porosity of 25% or less, preferably 20% or less, more preferably 15% or less, even more preferably 12% or less. When the open porosity is more than 25%, the number of voids in the zirconia molded body overly increases. This causes the cooling solvent contaminated with glass material to permeate into the zirconia molded body during wet processing, and the zirconia molded body turns white upon firing, making it unusable as a dental prosthesis. The open porosity is preferably 5% or more, more preferably 6% or more, even more preferably 7% or more, particularly preferably 8% or more. When the open porosity is less than 5%, the binder cannot easily burn itself off, and may remain even after the debinding step (a step that burns the binder). In the present invention, "open pore" means a pore (void) that is open to the external surface, as specified by JIS Z 8890:2017. In the present invention, "open porosity" means a percentage of the volume of open pore portions in the total volume (external volume) of a zirconia molded body. The method of open porosity measurement used in the present invention will be described in detail in the EXAMPLES section below.

A zirconia molded body of the present invention comprises zirconia (zirconium oxide; $ZrO_2$). In the present invention, "zirconia molded body" refers to a molded body formed by a method such as press forming, injection molding, or stereolithography using various forms of zirconia, such as a powder, a granule, a paste, or a slurry, as the primary raw material, and "zirconia molded body" is in a state yet to be pre-sintered or sintered. That is, "zirconia molded body" distinguishes itself from zirconia pre-sintered body and zirconia sintered body in that it is a molded body that has not been fired.

A zirconia molded body of the present invention comprises a stabilizer capable of inhibiting a phase transformation of zirconia. The stabilizer is preferably one capable of forming partially stabilized zirconia. Examples of the stabilizer include oxides such as calcium oxide (CaO), magnesium oxide (MgO), yttrium oxide ($Y_2O_3$) (hereinafter, referred to as "yttria"), cerium oxide ($CeO_2$), scandium oxide ($Sc_2O_3$), niobium oxide ($Nb_2O_5$), lanthanum oxide ($La_2O_3$), erbium oxide ($Er_2O_3$), praseodymium oxide ($Pr_6O_{11}$), samarium oxide ($Sm_2O_3$), europium oxide ($Eu_2O_3$), and thulium oxide ($Tm_2O_3$). The stabilizer may be used alone, or two or more thereof may be used in combination. The content of the stabilizer in a zirconia molded body of the present invention can be measured using a technique, for example, such as inductively coupled plasma (ICP) emission spectral analysis or X-ray fluorescence analysis. The content of the stabilizer in a zirconia molded body of the present invention is preferably 0.1 to 18 mol%, more preferably 1 to 15 mol%, even more preferably 2 to 8 mol% relative to the total mole of zirconia and stabilizer.

In view of the strength and translucency of a zirconia sintered body to be produced, the zirconia molded body preferably comprises yttria as the stabilizer. The yttria content is preferably 3 mol% or more, more preferably 3.5 mol% or more, even more preferably 3.8 mol% or more, particularly preferably 4.0 mol% or more relative to the total mole of zirconia and yttria. The zirconia sintered body can have increased translucency with an yttria content of 3 mol% or more. The yttria content is preferably 7.5 mol% or less, more preferably 7.0 mol% or less, even more preferably 6.5 mol% or less, particularly preferably 6.0 mol% or less relative to the total mole of zirconia and yttria. A decrease in the strength of the zirconia sintered body obtained can be reduced with an yttria content of 7.5 mol% or less.

A zirconia molded body of the present invention comprises a binder. In a zirconia molded body of the present invention, the binder content relative to the total mass of zirconia, stabilizer, and binder is preferably 3 to 10 mass%, more preferably 4 to 9 mass%, even more preferably 5 to 8 mass%, particularly preferably 6 to 8 mass% because the zirconia molded body can have the predetermined open porosity and strength with the binder content falling in these ranges. When the binder content is less than 3 mass%, the number of voids in the zirconia molded body overly increases. This may result in the cooling solvent contaminated with glass material permeating into the zirconia molded body during wet processing, and the zirconia molded body may turn white upon firing, making it unusable as a dental prosthesis. A binder content of more than 10 mass% may result in a granule that is too hard. This may seriously impair formability, and may cause trouble burning off the binder and removing it in the debinding step.

The binder may be a known binder commonly used for the production of a zirconia powder or a zirconia molded body. Specific examples of such binders include polyvinyl alcohol, methyl cellulose, carboxymethyl cellulose, polyvinyl butyrate, waxes (e.g., paraffin wax), and acrylic binders. Preferred are acrylic binders having a carboxyl group or derivatives thereof (for example, salts, particularly ammonium salts) within the molecule. Examples of the acrylic binders include polyacrylic acid, polymethacrylic acid, an acrylic acid copolymer, a methacrylic acid copolymer, and derivatives thereof. The binder may be used alone, or two or more thereof may be used in combination. An appropriate binder(s) may be selected according to the open porosity to be set.

A zirconia molded body of the present invention has a water absorbency of preferably 7% or less, more preferably 5% or less, even more preferably 4% or less, particularly preferably 3% or less. When the water absorbency is more than 7%, the cooling solvent contaminated with glass material may greatly permeate into the zirconia molded body during wet processing, and the zirconia molded body may turn white upon firing, making it unusable as a dental prosthesis. The method of measurement of water absorbency used in the present invention will be described in detail in the EXAMPLES section below. The water absorbency can be adjusted by appropriately selecting parameters such as the content and type of binder, and the applied molding pressure.

It is preferable in a zirconia molded body of the present invention that the zirconia is predominantly a monoclinic crystal system. In the present invention, " predominantly a monoclinic crystal system" means that the fraction $f_m$ of the monoclinic crystal system of zirconia is at least 50% of the total amount of all the crystal systems (monoclinic crystal system, tetragonal crystal system, and cubic crystal system) of the zirconia. The fraction $f_m$ of monoclinic crystal system is preferably 55% or more, more preferably 60% or more, even more preferably 70% or more, yet more preferably 75% or more, particularly preferably 80% or more, more particularly preferably 85% or more, most preferably 90% or more. In a zirconia molded body of the present invention, the peaks of tetragonal and cubic crystal systems may be essentially undetectable. That is, the fraction $f_m$ of the monoclinic crystal system may be 100%. The fraction $f_m$ of the monoclinic crystal system can be calculated from the mathematical expression (1) below, using peaks in an X-ray diffraction (XRD) pattern by CuK$\alpha$ radiation. The predominant crystal system in the zirconia molded body is a potential contributing factor that raises the shrinkage temperature and shortens the firing time.

[Math. 1]

$$f_m (\%) = \frac{l_m(111) + l_m(11-1)}{l_m(111) + l_m(11-1) + l_t(111) + l_c(111)} \times 100 \qquad (1)$$

In the mathematical expression (1), $I_m(111)$ and $I_m(11\text{-}1)$ represent the peak intensities of the (111) plane and (11-1) plane, respectively, of the monoclinic crystal system of zirconia. $I_t(111)$ represents the peak intensity of the (111) plane of the tetragonal crystal system of zirconia. $I_c(111)$ represents the peak intensity of the (111) plane of the cubic crystal system of zirconia.

In a zirconia molded body of the present invention, it is preferable that at least a part of zirconia crystals exist as monoclinic crystals by the presence of the stabilizer. To be more specific, it is preferable that at least a part of the stabilizer be present in an undissolved form in the zirconia as a solid solution. Whether a part of the stabilizer is undissolved in the zirconia as a solid solution can be determined by analyzing an XRD pattern, for example. The presence of a peak derived from the stabilizer in the XRD pattern of the zirconia molded body means that at least a part of the stabilizer is present in an undissolved form in the zirconia as a solid solution in the zirconia molded body. A peak derived from the stabilizer is basically not observable in the XRD pattern when the stabilizer has fully dissolved as a solid solution. It is, however, possible, depending on the crystal state or other conditions of the stabilizer, that the stabilizer may not be dissolved in zirconia as a solid solution even when the stabilizer does not produce a peak in the XRD pattern. When the crystal system of zirconia is predominantly tetragonal and/or cubic and there is no peak attributed to the stabilizer in the XRD pattern, the stabilizer can be thought of having been dissolved in zirconia as a solid solution for the most part, basically completely. In a zirconia molded body of the present invention, it is not required to fully dissolve the stabilizer in the zirconia as a solid solution. In the present invention, "to dissolve the stabilizer as a solid solution" means that, for example, the elements (atoms) contained in the stabilizer are dissolved in the zirconia as a solid solution.

A zirconia molded body of the present invention may optionally comprise an additive(s). Examples of the additive include colorants (including pigments, complex pigments, and fluorescent pigments), alumina ($Al_2O_3$), titanium oxide ($TiO_2$), and silica ($SiO_2$). The additive may be used alone, or two or more thereof may be used as a mixture.

Examples of the pigments include oxides of at least one element selected from the group consisting of Ti, V, Cr, Mn, Fe, Co, Ni, Zn, Y, Zr, Sn, Sb, Bi, Ce, Pr, Sm, Eu, Gd, Tb, and Er (specifically, for example, NiO, $Cr_2O_3$), preferably oxides of at least one element selected from the group consisting of Ti, V, Cr, Mn, Fe, Co, Ni, Zn, Y, Zr, Sn, Sb, Bi, Ce, Pr, Sm, Eu, Gd, and Tb, more preferably oxides of at least one element selected from the group consisting of Ti, V, Cr, Mn, Fe, Co, Ni, Zn, Y, Zr, Sn, Sb, Bi, Ce, Sm, Eu, Gd, and Tb. A zirconia molded body of the present invention may be a zirconia molded body that does not contain erbium oxide ($Er_2O_3$). Examples of the complex pigments include composite oxides, for example, such as $(Zr,V)O_2$, $Fe(Fe,Cr)_2O_4$, $(Ni,Co,Fe)(Fe,Cr)_2O_4{\cdot}ZrSiO_4$, and $(Co,Zn)Al_2O_4$. Examples of the fluorescent pigments include $Y_2SiO_5{:}Ce$, $Y_2SiO_5{:}Tb$, $(Y,Gd,Eu)BO_3$, $Y_2O_3{:}Eu$, YAG:Ce, $ZnGa_2O_4{:}Zn$, and $BaMgAl_{10}O_{17}{:}Eu$.

In view of ease of milling and machinability, it is preferable that a zirconia molded body of the present invention satisfy a specific range of biaxial flexural strength. Specifically, a zirconia molded body of the present invention has a biaxial flexural strength is preferably 7 to 30 MPa, more preferably 8 to 25 MPa, even more preferably 9 to 19 MPa. The biaxial flexural strength can be measured using the method described in the EXAMPLES section below.

A method of production of a zirconia molded body of the present invention is not particularly limited, as long as the present invention can produce its effects. In an example method, zirconia, a stabilizer, and a binder are mixed wet in water to form a slurry, and the slurry is dried to granulate. The granulated material can then be pressed into a zirconia molded body. The binder may be added to a pulverized slurry of a zirconia powder and a stabilizer after the slurry is formed by adding a primary powder of a zirconia powder-stabilizer mixture to water. In this case, a zirconia molded body can be obtained by drying the slurry after the addition of a binder, and pressing the resultant granulated material (secondary powder) into a zirconia molded body. Any known method can be used for press forming of the granulated material, and the method may include, for example, a uniaxial press forming step and/or a cold isostatic pressing (CIP) step. Preferably, the uniaxial press forming step may be a process in which zirconia is filled into a pressure mold (die) of a desired size, and is uniaxially pressed by applying pressure with an upper and a lower punch. Here, the applied pressured is optimized as appropriate according to the size, open porosity, water absorbency, biaxial flexural strength, and zirconia particle size desired for the molded body, and is typically 10 MPa to 1,000 MPa. By increasing the applied molding pressure of the method, the zirconia molded body produced can have tighter voids, allowing a smaller open porosity to be set.

Another embodiment of the present invention may be a zirconia sintered body obtained after main firing of the zirconia molded body. With a zirconia molded body of the present invention, a zirconia sintered body (for example, a dental prosthesis) having excellent translucency can be conveniently produced in a single main firing step, without undergoing the complicated process of pre-sintering a zirconia molded body in a temperature range of from about 800 to 1,200°C, cooling the resulting pre-sintered body, and firing the pre-sintered body again after optionally processing the pre-sintered body (for example, by milling) into a desired shape (for example, a shape of a crown). Preferably, the main firing (sintering) of the zirconia molded body includes a debinding step (a step that burns the binder) of heating the zirconia molded body in a temperature range of 800°C or less before the maximum firing temperature is reached.

The main firing (sintering) of zirconia molded body and dental mill blank may be a step that uses a common dental porcelain furnace. The dental porcelain furnace may be a commercially available product. Examples of such commercially available porcelain furnaces include Noritake KATANA® F-1N and Noritake KATANA® F-2 (both are products from SK Medical Electronics Co., Ltd.). The zirconia molded body is held in a dental porcelain furnace for preferably 1 to 140 minutes. Preferably, the sintering process has a maximum firing temperature of 1,400 to 1,600°C, though the sintering temperature is not particularly limited. In the case of short main firing, the zirconia molded body is held in the furnace for preferably less than 30 minutes, more preferably at most 20 minutes, even more preferably at most 15 minutes at the maximum firing temperature. With the present invention, a decrease of the translucency of a zirconia sintered body upon firing can be reduced even when the cooling solvent used for wet processing of a dental CAD/CAM system is contaminated, irrespective of whether the firing is ordinary firing or short firing.

Yet another embodiment of the present invention may be a dental mill blank comprising the zirconia molded body. A zirconia molded body of the present invention is usable as a dental material, particularly preferably as a dental mill blank. In certain embodiments, a dental mill blank of the present invention may be a dental mill blank processed from the zirconia molded body without firing. The dental mill blank is optionally processed (for example, cut or milled) into the desired size and shape (for example, a disc shape or a cuboidal shape), and is shipped after surface polishing. Preferably, a dental mill blank of the present invention is formed into a suitable size that can be accommodated by a commercially available dental CAD/CAM system, or a dental mill blank of the present invention is preferably processed after being formed. Preferably, a dental mill blank of the present invention is formed into, for example, a prism shape measuring 40 mm × 20 mm × 15 mm, suited for fabrication of single bridges, a prism shape measuring 17 mm × 10 mm × 10 mm, suited for fabrication of inlays and onlays, a prism shape measuring 14 mm × 18 mm × 20 mm, suited for fabrication of full crowns, or a disc shape measuring 100 mm in diameter and 10 to 28 mm in thickness, suited for fabrication of long-span bridges and denture bases. However, the size of dental mill blank is not limited to these. Because of the increased amount of binder in the zirconia molded body, a dental mill blank of the present invention has improved strength, and can be properly milled with a common dental CAD/CAM system, before firing.

Examples of dental prostheses that can be produced by firing a dental mill blank of the present invention include crown restorations such as inlays, onlays, veneers, crowns, and bridges. Other examples include abutment teeth, dental posts, dentures, denture bases, and implant parts (fixtures and abutments). Preferably, for example, a commercially available dental CAD/CAM system is used for milling. Examples of such a CAD/CAM system include the CEREC system manufactured by Dentsply Sirona, and the KATANA® system manufactured by Kuraray Noritake Dental Inc.

A zirconia molded body of the present invention can be used also in applications other than dental use. Examples of such applications include electronic materials (such as sealing materials, and materials for forming laminates), common general-purpose composite material members, architectural parts, and components of electrical appliances, home appliances, and toys.

The present invention encompasses combinations of the foregoing features, provided that such combinations made in various forms within the technical idea of the present invention can produce the effects of the present invention.

EXAMPLES

The following describes the present invention in greater detail by way of Examples. It should be noted that the present invention is in no way limited by the following Examples, and various changes may be made by a person with ordinary skill in the art within the technical idea of the present invention.

Preparation of Raw Material Zirconia Powder

A raw material powder used to produce zirconia molded bodies of Examples and Comparative Examples was prepared in the following manner. First, a zirconia powder of about 100% monoclinic crystal system was mixed with an yttria powder to prepare a mixture having an yttria content of 6 mol% relative to the total mole of zirconia and yttria. The mixture was added to water to prepare a slurry, and pulverized and mixed wet with a ball mill until an average particle diameter of 0.13 $\mu$m or less was achieved. After pulverization, the slurry was dried with a spray dryer, and the resulting powder was fired at 950°C for 2 hours to prepare a powder (primary powder). The average particle diameter can be determined by using a laser diffraction scattering method. Specifically, for example, the average particle diameter can be measured by volume using a laser diffraction scattering method with a laser diffraction particle size distribution analyzer (SALD-2300, manufactured by Shimadzu Corporation), using a 0.2% sodium hexametaphosphate aqueous solution used as dispersion medium.

The primary powder was then added to water to prepare a slurry, and the slurry was pulverized and mixed wet with a ball mill until an average particle diameter of 0.13 $\mu$m or less was achieved. After pulverization, a binder (an acrylic binder SA-200 manufactured by Japan Coating Resin Co., Ltd.) was added to the slurry in such an amount that the resulting mixture had the binder content shown in Table 1. The mixture was then dried with a spray dryer to prepare a granule (secondary powder). The granule was used as a raw material powder to produce zirconia molded bodies, as follows.

Preparation of Zirconia Molded Body

The raw material powder was charged into a cylindrical mold of about 15 mm diameter. Here, the zirconia powder was used in such an amount that the zirconia sintered body after sintering had a thickness of 1.3 to 1.5 mm. The zirconia powder was then pressed under a 100 kN load to obtain a zirconia molded body.

Measurement of Open Porosity and Water Absorbency of Zirconia Molded Body

The following formulae were used to calculate the open porosity and water absorbency of the zirconia molded body of each Example and Comparative Example, using a dry mass (M1), an underwater mass (M2), and a hydrous mass (M3) measured by the Archimedes method.

$$\text{Open porosity (\%)} = 100 \times (M3 - M1)/(M3 - M2)$$

$$\text{Water absorbency (\%)} = 100 \times (M3 - M1)/M1$$

For the measurements by the Archimedes method, an electronic balance (ML204/02 manufactured by Mettler-Toledo Inc.) fitted with a density measurement kit (ML-DNY-43) was used. The underwater mass (M2) is the mass of a zirconia molded body measured in a suspended state in water after the zirconia molded body, dried, was immersed in water for 1 hour. The hydrous mass (M3) is a mass measured after a zirconia molded body is measured for underwater mass (M2) by being dried and immersed in water for 1 hour, and represents the mass of the zirconia molded body holding water in open pores of its sample after being taken out of water and having water droplets removed from the surface. Table 1 shows the measured open porosity and water absorbency.

Evaluation of Translucency of Zirconia Sintered Body

The zirconia molded body of each Example and Comparative Example was subjected to main firing including a debinding step, using a furnace (Noritake KATANA® F-1 manufactured by SK medical electronics Co., Ltd.). Specifically, the zirconia molded body was retained at 1,550°C for 15 minutes to prepare a specimen of zirconia sintered body. The specimen was polished from both sides at #600 to prepare a zirconia sintered body, 1.2 mm thick, and the translucency was evaluated using the following method (n = 3). Translucency was measured with a Crystaleye (a dental color-analysis device manufactured by Olympus Corporation; a 7-band LED light source). First, a first L* value is obtained by measuring an L* value of the L*a*b* color system (JIS Z 8781-4:2013 Color Measurements - Part 4: CIE 1976 L*a*b* color space) for a specimen against a white background (underlay) (the opposite side of the specimen from the measurement device is white). Secondly, from the same specimen used for the measurement of first L* value, a second L* value is obtained by measuring an L* value of the L*a*b* color system against a black background (underlay) (the opposite side of the specimen from the measurement device is black).

In the present invention, translucency, denoted as ∆L*, is the difference between a first L* value and a second L* value (∆L* = first L* value - second L* value). Larger values of ∆L* mean higher translucency, and smaller values of ∆L* mean lower translucency. The black and white backgrounds (underlays) used for the chromaticity measurement may use the hiding-power test paper used for coating measurements in JIS K 5600-4-1:1999. Table 1 shows the translucencies (mean values) of zirconia sintered bodies measured after short firing (Translucency ($\Delta$L*) after firing by ordinary method).

Evaluation of Translucency of Fired Zirconia Sintered Body After Immersion in Contaminated Cooling Solvent

The translucency of a zirconia sintered body was evaluated using the following method, in order to confirm the influence of a contaminated cooling solvent on translucency of a zirconia sintered body. First, a glass material (e.max; Ivoclar Vivadent) was milled wet 25 times with a dry-wet milling machine (Cerec MC XL; manufactured by Dentsply Sirona) to contaminate a cooling solvent (processing liquid; a hydrophilic solvent with main component water; DENTATEC manufactured by Dentsply Sirona under this trade name). In order to reproduce a situation where a zirconia molded body is contacted by a contaminated processing liquid during wet milling, the contaminated processing liquid was filled into a container, and a specimen of the zirconia molded body of each Example and Comparative Example was immersed therein for 15 minutes-about the same length of time used in actual processing of a dental prosthesis-to allow the processing liquid to permeate the specimen (n = 3). This was followed by main firing, in which the zirconia molded body was retained at 1,550°C for 15 minutes using a furnace (Noritake KATANA® F-1 manufactured by SK medical electronics Co., Ltd.). The resulting fired specimen of zirconia sintered body was then measured for translucency $\Delta$L* using the method described above. The results are presented in Table 1 as mean values.

Evaluation of Strength of Zirconia Molded Body

A zirconia molded body of each Example and Comparative Example, prepared separately from the zirconia molded bodies produced above, was formed into a sample for strength measurement. The sample, measuring about 15 mm in diameter and 1.2 mm in thickness, was measured for biaxial flexural strength with a universal testing machine (manufactured by Instron) with the crosshead speed set at 0.5 mm/min, according to ISO 6872:2015 (n = 5). The results are presented in Table 1 as mean values.

[Table 1]

| | Example 1 | Example 2 | Example 3 | Example 4 | Example 5 | Comparative Example 1 | Comparative Example 2 |
|---|---|---|---|---|---|---|---|
| Binder Content (%) | 4 | 6 | 7 | 8 | 10 | 2 | 12 |
| Open Porosity (%) | 21 | 14 | 9 | 10 | 17 | 28 | 26 |
| Water Absorbency (%) | 5.8 | 4 | 3.7 | 2.8 | 5.8 | 7.9 | 8.5 |
| Biaxial flexural strength (MPa) | 14 | 15 | 13 | 15 | 11 | 6 | 2 |
| Translucency ($\Delta$L*) after firing by ordinary method | 16.0 | 16.6 | 16.2 | 15.7 | 14.2 | 16.0 | 7.8 |
| Translucency ($\Delta$L*) after firing following immersion in contaminated cooling solvent | 15.4 | 16.3 | 16.3 | 15.6 | 14.0 | 11.1 | 6.6 |

The zirconia molded bodies of Examples 1 to 5 had an open porosity of 25% or less, and retained their translucency after firing because of reduced permeation of contaminated cooling solvent into the voids even after immersion in contaminated cooling solvent. In contrast, the zirconia molded body of Comparative Example 1, with a high open porosity of 28%, had a much smaller translucency in the sintered body as a result of permeation of contaminated cooling solvent into the voids, and the strength was also weaker. The zirconia molded body of Comparative Example 2 had an open porosity greater than 25%. This, combined with the excessively large amount of binder, caused deterioration of formability, leading to a failure to sufficiently crush the granules during pressing. In addition, the translucency after firing by an ordinary method was poor, and was even poorer when the zirconia molded body was immersed in contaminated cooling solvent. The strength was also low, even when compared to Comparative Example 1.

The numeric ranges given in this specification should be construed such that all numerical values and ranges falling within the ranges specified herein are specifically recited in the specification, even in the absence of specific recitations.

INDUSTRIAL APPLICABILITY

A zirconia molded body of the present invention can be suitably used in a variety of applications, including dental products for fabrication of articles such as dental prostheses.

**Claims**

1. A zirconia molded body

   comprising

      zirconia;
      a stabilizer capable of inhibiting a phase transformation of zirconia; and
      a binder, and

   having an open porosity of 25% or less.

2. The zirconia molded body according to claim 1, wherein the open porosity is 5% or more.

3. The zirconia molded body according to claim 1 or 2, wherein the binder is present in an amount of 3 to 10 mass% relative to a total mass of the zirconia, the stabilizer, and the binder.

4. The zirconia molded body according to claim 3, wherein the binder is present in an amount of 5 to 8 mass%.

5. The zirconia molded body according to any one of claims 1 to 4, wherein the zirconia molded body has a water absorbency of 7% or less.

6. The zirconia molded body according to any one of claims 1 to 5, wherein the zirconia is predominantly a monoclinic crystal system.

7. The zirconia molded body according to any one of claims 1 to 6, wherein at least a part of the stabilizer is undissolved in the zirconia as a solid solution.

8. The zirconia molded body according to any one of claims 1 to 7, wherein the stabilizer is yttria.

9. The zirconia molded body according to claim 8, wherein the yttria is present in an amount of 3 mol% to 7.5 mol% relative to a total mole of the zirconia and the yttria.

10. The zirconia molded body according to any one of claims 1 to 9, wherein the binder is at least one selected from the group consisting of polyvinyl alcohol, methyl cellulose, carboxymethyl cellulose, polyvinyl butyrate, a wax, and an acrylic binder.

11. The zirconia molded body according to any one of claims 1 to 10, wherein the zirconia molded body has a biaxial flexural strength of 7 to 30 MPa.

12. A dental mill blank comprising a zirconia molded body of any one of claims 1 to 11.

<table>
<tr><td colspan="2" align="center">**INTERNATIONAL SEARCH REPORT**</td><td colspan="2">International application No.<br>PCT/JP2020/039157</td></tr>
</table>

**A.    CLASSIFICATION OF SUBJECT MATTER**

C04B 35/486(2006.01)i; A61C 5/77(2017.01)i; A61C 13/083(2006.01)i; A61K
6/818(2020.01)i
FI: C04B35/486; A61K6/818; A61C13/083; A61C5/77
According to International Patent Classification (IPC) or to both national classification and IPC

**B.    FIELDS SEARCHED**

Minimum documentation searched (classification system followed by classification symbols)
C04B35/486; A61C5/77; A61C13/083; A61K6/818

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X<br>Y | WO 2014/181828 A1 (KURARAY NORITAKE DENTAL INC.)<br>13 November 2014 (2014-11-13) paragraphs [0126]-<br>[0129], [0201] | 1-9, 11-12<br>10 |
| X<br><br>Y | JP 2013-49616 A (TOSOH CORP.) 14 March 2013 (2013-03-14) paragraphs [0067]-[0072], [0122] | 1-3, 5-9, 11-12<br><br>10 |
| X<br>Y | WO 2018/056330 A1 (KURARAY NORITAKE DENTAL INC.)<br>29 March 2018 (2018-03-29) paragraphs [0011],<br>[0065]-[0066], [0092]-[0095], [0150], [0156]-<br>[0164] | 1-2, 5-12<br>10 |
| X<br>Y | WO 2011/021698 A1 (NORITAKE CO., LTD.) 24 February<br>2011 (2011-02-24) paragraphs [0123]-[0127], [0226] | 1-5, 7-12<br>10 |

☐    Further documents are listed in the continuation of Box C.        ☒    See patent family annex.

| | |
|---|---|
| *    Special categories of cited documents:<br>"A"    document defining the general state of the art which is not considered<br>to be of particular relevance<br>"E"    earlier application or patent but published on or after the international<br>filing date<br>"L"    document which may throw doubts on priority claim(s) or which is<br>cited to establish the publication date of another citation or other<br>special reason (as specified)<br>"O"    document referring to an oral disclosure, use, exhibition or other means<br>"P"    document published prior to the international filing date but later than<br>the priority date claimed | "T"    later document published after the international filing date or priority<br>date and not in conflict with the application but cited to understand<br>the principle or theory underlying the invention<br>"X"    document of particular relevance; the claimed invention cannot be<br>considered novel or cannot be considered to involve an inventive<br>step when the document is taken alone<br>"Y"    document of particular relevance; the claimed invention cannot be<br>considered to involve an inventive step when the document is<br>combined with one or more other such documents, such combination<br>being obvious to a person skilled in the art<br>"&"    document member of the same patent family |

| Date of the actual completion of the international search<br>    08 December 2020 (08.12.2020) | Date of mailing of the international search report<br>    15 December 2020 (15.12.2020) |
|---|---|
| Name and mailing address of the ISA/<br>    Japan Patent Office<br>    3-4-3, Kasumigaseki, Chiyoda-ku,<br>    Tokyo 100-8915, Japan | Authorized officer<br><br><br>Telephone No. |

Form PCT/ISA/210 (second sheet) (January 2015)

**INTERNATIONAL SEARCH REPORT**
Information on patent family members

| International application No. |
|---|
| PCT/JP2020/039157 |

| Patent Documents referred in the Report | Publication Date | Patent Family | Publication Date |
|---|---|---|---|
| WO 2014/181828 A1 | 13 Nov. 2014 | US 2016/0074142 A1 paragraphs [0129]-[0132], [0175] EP 2995434 A CN 105189067 A KR 10-2016-0007574 A | |
| JP 2013-49616 A | 14 Mar. 2013 | US 2014/0227654 A1 paragraphs [0092]-[0097], [0146] WO 2013/018728 A1 EP 2738147 A1 CN 103732559 A KR 10-2014-0056168 A | |
| WO 2018/056330 A1 | 29 Mar. 2018 | EP 3517518 A1 paragraphs [0011], [0065]-[0066], [0090]-[0093], [0148], [0154]-[0162] CN 109689593 A KR 10-2019-0047701 A | |
| WO 2011/021698 A1 | 24 Feb. 2011 | US 2012/0214661 A1 paragraphs [0147]-[0151], [0210] EP 2468699 A1 CN 102482162 A KR 10-2012-0062705 A | |

Form PCT/ISA/210 (patent family annex) (January 2015)

**EP 4 046 977 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019108289 A